# EUROPÄISCHE PATENTSCHRIFT

(11) **EP 0 820 256 B1**
(45) Veröffentlichungstag und Bekanntmachung des Hinweises auf die Patenterteilung: **24.07.2002**
(21) Anmeldenummer: 96909046.3
(22) Anmeldetag: 15.04.1996
(51) Int. Cl.: A61B 17/00, A61C 1/18, B23B 31/22

(54) **KUPPLUNG FÜR ROHRSCHAFTINSTRUMENTE**
COUPLING FOR TUBULAR-SHAFT INSTRUMENTS
ACCOUPLEMENT POUR INSTRUMENTS TUBULAIRES

(30) Priorität: 13.04.1995 DE 19514098
(43) Veröffentlichungstag der Anmeldung: 28.01.1998
(73) Patentinhaber: Karl Storz GmbH & Co. KG, 78532 Tuttlingen (DE)
(72) Erfinder: DITTRICH, Horst, D-78194 Immendingen (DE); BACHER, Uwe, D-78532 Tuttlingen (DE); SAUER, Michael, D-78532 Tuttlingen (DE)
(74) Vertreter: Münich, Wilhelm, Dr.
(86) Internationale Anmeldenummer: DE9600650
(87) Internationale Veröffentlichungsnummer: WO9632068

(56) Entgegenhaltungen:
- DE-A- 4 311 161
- FR-A- 2 465 467
- US-A- 4 403 959
- US-A- 4 577 875

## Beschreibung

### Technisches Gebiet

Die Erfindung bezieht sich auf eine Kupplung für Rohrschaftinstrumente, wobei mit der Kupplung das Instrumententeil mit dem Schaftrohr lösbar fest verbindbar und die Kupplung an dem Schaftrohr oder dem Instrumententeil anbringbar ist.

### Stand der Technik

In der Endoskopie und insbesondere in der Mikrochirurgie ist es häufig erforderlich, ein Endoskop, einen Trokar, eine Zange, eine Schere oder ein anderes Instrument in einen Schaft einzusetzen, der beispielsweise ein Universalschaft, eine Trokarhülse oder dgl. sein kann. Dabei ist es für viele Anwendungen erforderlich, dass das in den Schaft eingesetzte Instrument im Einsatz fest mit dem Schaft verbunden ist, und bei Bedarf wieder gelöst werden kann. Zur Herstellung dieser festen Verbindung ist in der Regel eine Kupplung vorgesehen. Diese Kupplungen werden von den einzelnen Herstellern in den unterschiedlichsten Ausführungen gefertigt. In der Regel weisen diese Kupplungen Bajonettelemente und einen Drehring auf, der zum Verriegeln und zum Entriegeln gegenläufig gedreht werden muss.

Unabhängig hiervon besteht ein zunehmender Bedarf an Instrumenten und insbesondere an Rohrschaftinstrumenten oder Endoskopen, die durch wenige Handgriffe zerlegbar sind, um die Instrumente vor und/oder nach einer Operation zu reinigen und zu sterilisieren, ohne dass in schwer zugänglichen, beispielsweise verwinkelten Ecken der Instrumente Rückstände verbleiben. Auch in diesem Falle ist der Einsatz von Kupplungen erforderlich, durch die erst die leichte Zerlegbarkeit von Instrumenten und insbesondere von Rohrschaftinstrumenten sichergestellt wird.

Weiterhin wird es häufig gewünscht, wenigstens die Teile von Instrumenten, die mit dem Körper des Patienten in Berührung kommen, nur einmal zu verwenden. Um die häufig sehr komplexen Instrumente dennoch so günstig wie möglich anbieten zu können, ist es sinnvoll, die Teile, die nicht direkt mit dem Körper des Patienten in Berührung kommen, als mehrfach verwendbare Teile auszugestalten. Auch in diesem Fall ist es erforderlich, um Instrumententeile schnell, einfach und sicher auszutauschen, Kupplungen oder dgl. vorzusehen.

Aus der FR-A-2465467 ist ein zehnärztliches Hendstück mit einer Drehhülse zum lösen der Kupplung durch Drehung, mit einem Schaftaufnahmeteil und mit einer nach außen bewegbaren Rastkugel bekannt.

Aus der DE 39 34 610 A1 ist eine Schnellkupplung für chirurgische Instrumente bekannt. Diese Schnellkupplung weist einen Einsteckzapfen mit mindestens einem Rücksprung in seiner Umfangsfläche auf. Ein Gehäuse der Schnellkupplung ist mit einer Einstecköffnung für den Einsteckzapfen versehen. In der Wand des Gehäuses ist mindestens ein radial und axial verschiebbar gehaltener Verriegelungskörper vorgesehen, der bei eingeschobenem Einsteckzapfen mit dem Rücksprung ausgerichtet ist. Ein auf dem Gehäuse zwischen zwei Endstellungen verschiebbar gelagerter Schieber liegt in einer ersten Endstellung an dem Verriegelungskörper an und schiebt diesen nach innen über die Innenwand der Einstecköffnung hinaus in diese ein. In der zweiten Endstellung gibt der Schieber den Weg für den Verriegelungskörper frei, so dass dieser so weit radial nach außen verschoben ist, dass er nicht mehr in die Einstecköffnung eintaucht. Aus dieser Druckschrift ist es allerdings nicht bekannt, bei einer Schnellkupplung eine um eine Schaftaufnahme angeordnete Drehhülse vorzusehen, mittels derer nur durch Drehen und nicht durch Verschieben das Lösen der Verbindung erfolgt.

Aus der EP 0 056 266 A1 ist ein Schnellwechselfutter bekannt, das zwischen Antriebsteilen und/oder Werkzeugen mit Mitnehmerbacken zur Drehmitnahme und Keileingriff zur lösbaren Längsverbindung insbesondere für chirurgische Instrumente verwendbar ist. Dabei ist eine als Antriebsteil dienende Verriegelungshülse mit einem radial verschiebbarem Verriegelungsteil versehen, welche von einem aufgeschobenen Verriegelungsring umfasst ist, der mittels einer Verriegelungskurve durch Verdrehen des Verriegelungsteils zur Verriegelung mit dem eingeführten Antriebsteil oder Werkzeug bringt, wobei in den Verriegelungsring einerseits eine Ringfeder eingreift, welche andererseits in einen Verschlussring greift, der mittels eines Verschlussstifts an einer Öffnung an der Verriegelungshülse gegebenenfalls mit Ringeinsatz bis zu einer Ringnut einschiebt und unter Federwirkung gegenüber der Öffnung verriegelbar ist.

Dieses vorbekannte Schnellwechselfutter ist allerdings recht kompliziert aufgebaut und ermöglicht es dem Benutzer nicht, die Instrumententeile schnell, einfach und sicher auszutauschen. Darüber hinaus dürfte die Sterilisierung dieses Schnellwechselfutters schwierig sein.

Alle vorstehend genannten Kupplungen haben zusätzlich zu den vorstehend genannten Nachteilen den Nachteil, dass sowohl zum Verriegeln als auch zum Entriegeln der Kupplung eine extern von der Bedienungsperson bewusst herbeigeführte Manipulation erforderlich ist.

### Darstellung der Erfindung

Es ist Aufgabe der vorliegenden Erfindung, eine Kupplung für Rohrschaftinstrumente anzugeben, die es ermöglicht Teile von Rohrschaftinstrumenten schnell und einfach lösbar fest miteinander zu verbinden, wobei nach Verbindung dieser Teile eine sichere Handhabung ermöglicht wird. Insbesondere soll es die erfindungsgemäße Kupplung ermöglichen, die zu kuppelnden Teile miteinander zu verbinden, ohne dass eine bewusst herbeigeführte Manipulation an der Kupplung erforderlich wäre.

Eine erfindungsgemäße Lösung dieser Aufgabe ist in den unabhängigen Patentansprüchen angegeben. Weiterbildungen der vorliegenden Erfindung sind den abhängigen Ansprüchen zu entnehmen.

Die Erfindung geht von einer Kupplung für Rohrschaftinstrumente aus, die ein Schaftrohr mit einem Instrumententeil, wie beispielsweise einem Griff, lösbar fest verbindet, und die eine Drehhülse aufweist, die um ein Schaftaufnahmeteil angeordnet ist. Ferner ist eine Rasthülse, wie beispielsweise eine Kugelhülse vorgesehen, die zwischen Drehhülse und Schaftaufnahmeteil angeordnet ist und in axialer Richtung, die durch die Längsachse des Schaftrohres definiert ist, bewegbar ist, und zumindest ein Rastelement, wie eine Kugel aufnimmt, die sich nach außen, d.h. radial von der Längsachse des Schaftrohres weg bewegen lässt. Die Verbindung des Instrumententeils, das an seinem zur Kupplung reichenden Ende an seiner Außenfläche wenigstens einen Einstich aufweist, geschieht durch Einschieben dieses Endes des Instrumententeils in die Kupplung und Einrasten der Kugel bzw. der Kugeln in dem oder den Einstichen.

Durch diese Maßnahmen ist es möglich, ein Instrumententeil mit dem Schaftrohr schnell und einfach zu verbinden. Insbesondere hat die erfindungsgemäße Kupplung den Vorteil, dass zum Verriegeln der Kupplung die Drehhülse nicht betätigt werden muss. Das Verriegeln erfolgt vielmehr selbsttätig. Lediglich zum Entriegeln der Kupplung muss die Drehhülse gedreht werden.

Durch die konische Ausgestaltung des Einstichs am Instrumententeil wird es ermöglicht, dass das Rastelement, also beispielsweise die Kugel bzw. die Kugeln, die von der Kugelhülse aufgenommen sind, in dem Einstich einrasten, und somit eine entsprechend der Größe der Reibungskräfte und Rückstellkräfte feste Verbindung entsteht.

Beim Einführen des Intrumententeils wird das Rastelement bzw. werden die Rastelemente durch das eine Ende des Instrumententeils längs einer Schräge zwangsweise bewegt. Dadurch wird die Kugel aus dem Bereich, in den das Ende des Instrumententeils hineingeschoben wird, herausgedrängt, um anschließend durch entsprechende Rückstellkräfte zurückzuschnappen. Vorteilhafterweise ist die Schräge, die in der Drehhülse angeordnet sein kann, konisch.

Es ist weiterhin sehr vorteilhaft, wenn der Winkel, der durch die Achse und die Schräge definiert ist, größer ist als der Winkel der durch die Achse und den Einstich definiert ist. Dadurch wird der Raum für die Kugeln in axialer Richtung beim Einrasten der Kugeln in die Einstiche enger. Durch eine Rückstellkraft wird erreicht, dass die Kugeln in den enger werdenden Spalt gedrückt werden, sich verklemmen und die Kupplung auf dem Instrumententeil fest zentrieren. Es ist dabei sinnvoll, dass die Rückstellkraft die Kugelhülse und damit die Kugeln in Richtung ihrer Ausgangsposition drückt.
Vorteilhafterweise wird die Rückstellkraft durch eine Feder, wie eine Schrauben- oder Druckfeder bewegt, die eine definierte Kraft angeben. Durch eine entsprechende Vorrichtung ist es möglich, diese Kraft durch Änderung der Federkonstanten zu variieren. So ist es z.B. möglich als Druckfedern pneumatische Federn zu verwenden, deren pneumatischer Druck eingestellt werden kann.
Erfindungsgemäß geschieht das Verbinden der Kupplung mit dem Instrumententeil oder/und das Verbinden der Kupplung mit dem Schaftrohr wie folgt:
- der Instrumententeil wird in den Schaftaufnahmeteil geschoben und kommt mit mindestens einem Rastelement in Kontakt,
- durch weiteres Schieben des Instrumententeils wird das Rastelement bis zu einer zu der Ausgangslage am entferntesten liegenden Stellung verschoben,
- und durch weiteres Schieben des Instrumententeils bewegt sich das Rastelement wieder zurück in den Einstich des Instrumententeils.

Dort verklemmen sich die Kugeln und die Kupplung wird auf den Instrumententeil bzw. das Schaftrohr zentriert.

Die Entkupplung wird durch gleichzeitiges Drehen einer Drehhülse und Ziehen des Instrumententeils ermöglicht. Dazu ist es vorteilhaft, wenn eine in der Drehhülse angeordnete Aussparung, die zum Teil durch die schon beschriebene Schräge begrenzt ist, über den Umfang der Drehhülse unterschiedlich große Querschnitte aufweist.

Durch Verdrehen der Drehhülse relativ zur Kugelhülse dergestalt, dass die Aussparung über der Kugel bzw. den Kugeln einen größeren Querschnitt aufweist, erhalten diese die Möglichkeit, dem Druck durch das Ziehen des Instrumententeils und damit der übertragenen Kraft des jeweiligen Einstichs auf die dazu gehörige Kugel vertikal von der Längsachse auszuweichen. Dadurch wird der Instrumententeil lösbar.

Weiterhin ist es vorteilhaft, wenn eine Rückstellkraft die Drehhülse in Richtung ihrer Ausgangsposition drückt. Diese Rückstellkraft kann z.B. durch eine Spiralfeder erreicht werden. Sofern die Option gewünscht ist, dass die Rückstellkraft einstellbar ist, bietet sich auch für diese Feder eine pneumatische Feder an, deren pneumatischer Druck einstellbar ist.

Anstelle einer Rückstellkraft durch eine Feder oder zusätzlich dazu ist es möglich, durch einen lösbar festen Eingriff eines Stifts in ein Loch oder eine Nut die Drehung der Drehhülse zu verhindern, wenn der Instrumententeil oder das Schaftrohr in der Kupplung eingerastet ist.

Vorteilhafterweise wird die Kupplung für Rohrschaftinstrumente verwendet.

Eine weitere Lösung der Aufgabe ist durch eine Kupplung gegeben, die die folgenden Merkmale aufweist:
- eine Kugelhülse, die wenigstens eine Kugel aufnimmt, wobei die Kugel bzw. Kugeln nach außer bewegbar sind,
- eine Drehhülse, die eine Aussparung über ihren Umfang aufweist, die zumindest durch eine Schräge begrenzt ist und unterschiedlich in die Drehhülse hineinreicht, wobei die Kugelhülse in Richtung der Längsachse der Kupplung verschiebbar ist und wenigstens teilweise in Kontakt mit der Drehhülse ist und wobei die Kugel bzw. die Kugeln sowohl in Richtung der Längsachse der Kupplung mit der Kugelhülse mitbewegt werden und zusätzlich in etwa im rechten Winkel zur Längsachse der Kupplung bewegbar sind, wobei die Auslenkung der jeweiligen Kugel durch die Schräge und die in etwa im rechten Winkel zur Längsachse der Kupplung angeordnete Ausdehnung der Aussparung begrenzt ist.

Um eine lösbar feste Verbindung mit dieser Kupplung zwischen einem Intrumententeil oder/und einem Schaftrohr zu ermöglichen, ist am Ende des Instrumententeils bzw. Schaftrohrs zumindest ein Einstich vorgesehen, in den die Kugel bzw. bei mehreren Einstichen die Kugeln nach dem Aufstecken greifen.

Die Verbindung wird durch Rückstellkräfte von mindestens einer Federn und/oder durch formschlüssige Stifte aufrecht erhalten.

### Kurze Beschreibung der Zeichnung

Die Erfindung wird nachstehend anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
Fig. 1: in der Mitte einen Querschnitt durch eine erfindungsgemäße Kupplung, wobei links ein Teil eines Schaftrohrs dargestellt ist, sowie auf der linken Seite eine Ansicht der mittleren Figur von der linken Seite und auf der rechten Seite des Bildes eine Ansicht der mittleren Figur von der linken Seite, ohne Zeichnung der Abdeckhülse,
Fig. 2: einen Querschnitt der Kupplung aus Fig. 1 mit einem Teil eines Instrumententeils,
Fig. 3: links: wie Fig. 2 mit eingerastetem Instrumententeil; rechts: Schnitt wie links angegeben ohne Schaftaufnahme und Kugelhülse gezeichnet,
Fig. 4: eine Ausschnittszeichnung aus Fig. 3, und
Fig. 5: entsprechend Fig. 3 während der Entriegelung der Kupplung mit dem Instrumententeil.

### Darstellung von Ausführungsbeispielen

In den folgenden Figuren sind jeweils gleiche oder entsprechende Teile mit den selben Bezugszeichen bezeichnet, so dass auf eine erneute Vorstellung verzichtet wird, und lediglich die Abweichungen der in diesen Figuren dargestellten Ausführungsbeispiele gegenüber dem ersten Ausführungsbeispiel erläutert werden:

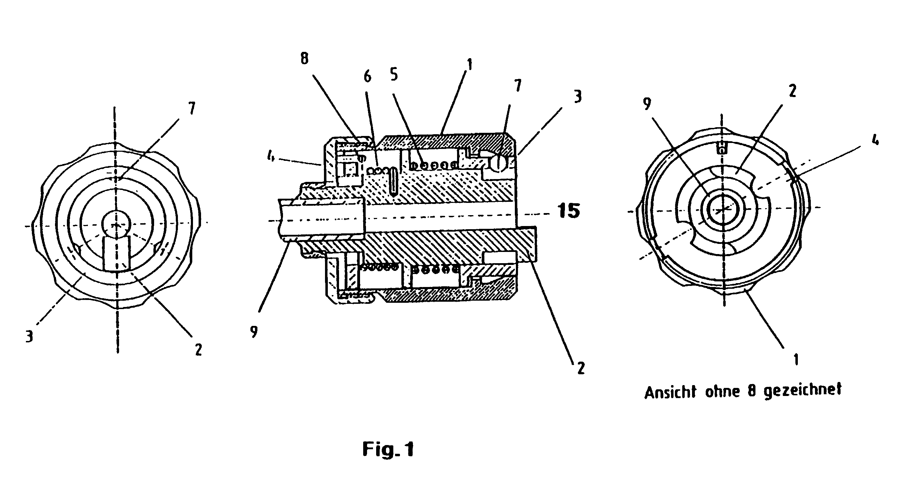

Fig. 1 zeigt eine Grundstellung einer erfindungsgemäßen Kupplung, deren Funktion das Verbinden und Lösen von Schaftrohr und Instrumententeil 10, als Beispiel mit einem Griff, bei zerlegbaren Rohrschaftinstrumenten ist, wobei die Kupplung in diesem Ausführungsbeispiel fest mit dem Schaftrohr 9 verbunden ist.

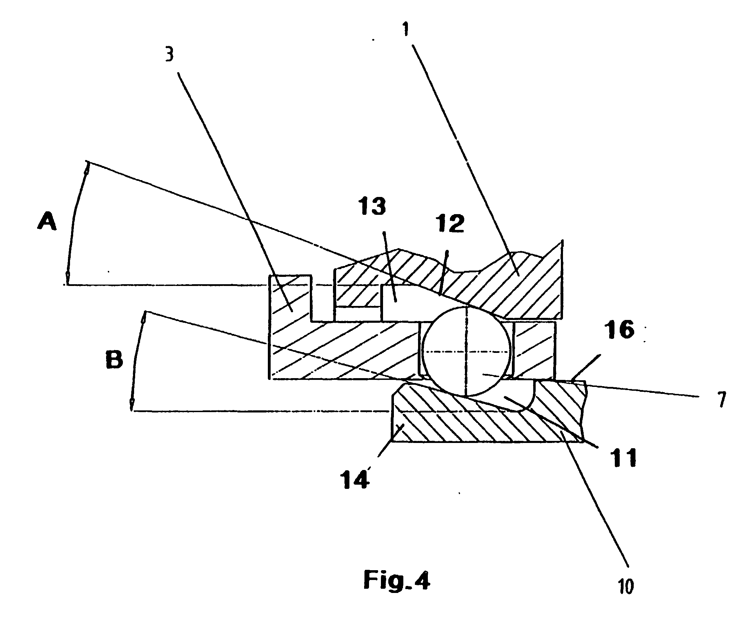

Selbstverständlich ist es aber auch möglich, die Kupplung nicht an dem Schaftrohr 9, sondern an dem Instrumententeil 10 anzuordnen.
Zu erkennen ist eine Drehhülse 1, die auf ihrem inneren Umfang eine Aussparung 13 aufweist. Diese Aussparung ist in Fig. 4 vergrößert dargestellt.

In die Aussparung 13 reicht eine Kugel 7, die in einer axialbeweglichen Kugelhülse 3 angeordnet ist.

Die axialbewegliche Kugelhülse wird durch eine Druckfeder 5 in ihre Aufgangslage gedrückt. Die Rückstellkraft einer Torsionsfeder 6 verhindert das ungewollte Verdrehen der Drehhülse 1.

Die Kupplung wird durch eine Anschlagscheibe 4 und eine Abdeckhülse gegen das Eindringen z.B. von Schmutz auf der Schaftrohrseite geschützt.

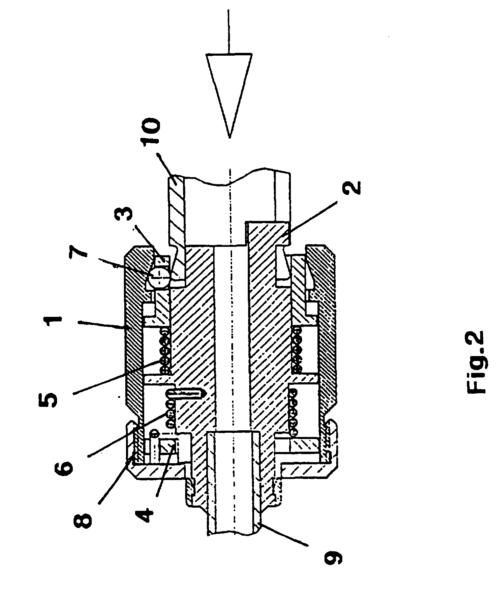

Zum Verbinden von Kupplung und Instrumententeil 10 wird die Kupplung axial auf den Instrumenteneil 10 geschoben. Dabei wird die Rückseite des Schaftaufnahmeteils 2 konzentrisch in die Bohrung des Instrumententeils 10 geführt. Beim Einführen des Instrumententeils drückt die Vorderkante des Instrumenten-teils (hier die Vorderkante eines Griffs) 14 auf die Kugeln 7 in der axialbeweglichen Kugelhülse 3 (siehe Fig. 2). Die Kugeln 7 liegen an der konischen Schräge der Drehhülsen 1 an.

Durch Überwinden der Federkraft der Druckfeder 5 wird die Kugelhülse 3 mit den Kugeln 7 zurückgedrückt und die Kugeln 7 gleiten an der konischen Schräge der Drehhülse 1 nach außen. Die Auslenkung der Kugeln entspricht der Durchmesserzunahme der konischen Fläche bzw. Schräge 12 in der Aussparung 13.

Dadurch wird das Lumen bzw. der Querschnitt, in das der Endbereich 14 des Instrumententeils hineingeschoben ist, größer und zwar so groß, dass der Endbereich des Instrumententeils weiter eingeführt werden kann als es durch die Ausgangslage der Kugeln gegeben wäre.

Ausgelöst durch die Rückstellkraft der Druckfeder 5 gleitet die Kugelhülse 3 wieder zurück in die Ausgangslage über den Außendurchmesser bzw. die Außenfläche 16 des Instrumententeils 10. Dabei werden die Kugeln 7 durch die konische Schräge 12 in der Drehhülse 1 wieder nach innen gedrückt und finden Platz in dem oder den konischen Einstichen 11 im Instrumententeil 10.

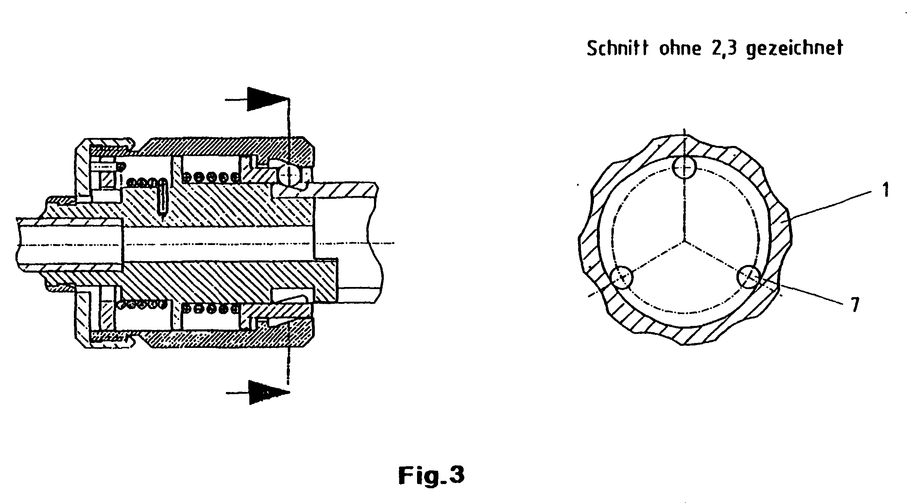

Der Instrumententeil 10 ist nun in der Kupplung eingerastet (siehe Fig. 3). Vorteilhaft ist hierbei, wie in Fig. 4 angedeutet, dass der Winkel A der konischen Schräge in der Drehhülse 1 größer ist als der Winkel B im Einstich am Instrumententeil 10. Der Raum für die Kugeln 7 wird also beim Einschnappen bzw. Einrasten in axialer Richtung geringer. Durch die Kraft der Druckfeder 5 wird erreicht, dass die Kugeln 7 in den enger werdenden Spalt gedrückt werden, sich verklemmen und damit die Kupplung auf dem Instrumententeil 10 fest zentriert wird.

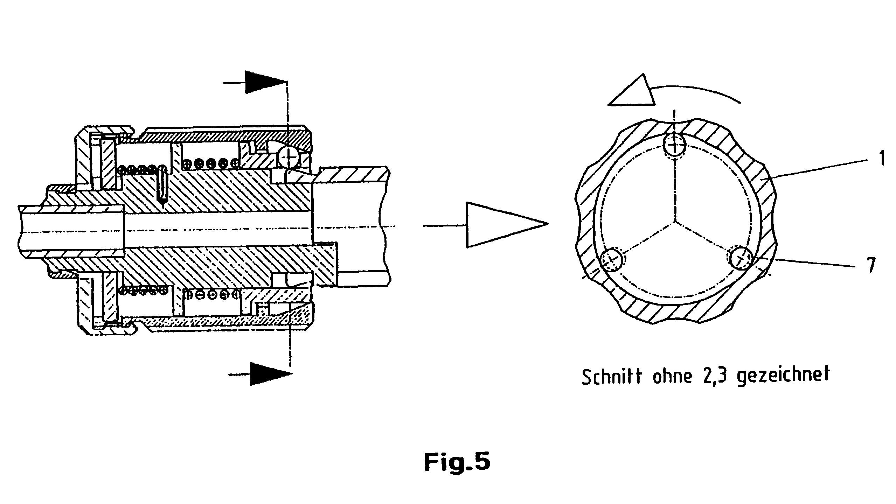

Zum Lösen der Kupplung vom Instrumententeil 10 wird gemäß Fig. 5 die Drehhülse 1 gegen die Verspannung der Torsionsfeder 6 z.B. nach links von der Rohrschaftseite aus gesehen gedreht und der Instrumententeil 10 herausgezogen. Durch das Drehen der Drehhülse 1 wird die Aussparung der exzentrisch konischen Schrägen, die über die Kugeln in Position gebracht werden, größer. Dadurch können sich die Kugeln 7, ausgelöst durch das Ziehen am Instrumententeil 10, nach außen von der Nut bzw. dem Einstich im Instrumententeil 10 wegbewegen und diese freigeben. Der Instrumententeil 10 ist nunmehr wieder von der Kupplung gelöst. Nach dem Loslassen der Drehhülse 1 dreht sich diese wieder durch die Rückstellkraft der Torsionsfeder 6 in die Grundstellung zurück.

## Patentansprüche

1. Kupplung für Rohrschaftinstrumente, die ein Instrumententeil (10) mit einem Schaftrohr (9), wobei das Instrumententeil (10) an einem zur Kupplung reichenden Ende (14) an einer Außenfläche (16) oder das Schaftrohr (9) wenigstens einen Einstich (11) aufweisen, lösbar fest verbindet und die Kupplung an dem Schaftrohr oder dem Instrumententeil anbringbar ist, mit
- einer Drehhülse (1) zum Lösen der Kupplung durch Drehung, die um ein Schaftaufnahmeteil (2) angeordnet ist, wobei
- eine in axialer Richtung, die durch die Längsachse (15) des Schaftrohrs (9) definiert ist, bewegbare Rasthülse (3) zwischen Drehhülse (1) und Schaftaufnahmeteil (2) angeordnet ist, die zumindest ein Rastelement (7) aufnimmt, das nach außen bewegbar ist, und
- die Kupplung das Instrumententeil (10) mit dem Schaftrohr (9) durch Einrasten des oder der Rastelemente (7) in dem oder den Einstichen (11) verbindet.

2. Kupplung nach Anspruch 1,
**dadurch gekennzeichnet, daß** der bzw. jeder Einstich (11) konisch ausgebildet ist.

3. Kupplung nach Anspruch 1 oder 2,
**dadurch gekennzeichnet, daß** die bewegbare Rasthülse (3) eine Kugelhülse und das oder die Rastelemente Kugeln (7) sind.

4. Kupplung nach einem der Ansprüche 1 bis 3,
**dadurch gekennzeichnet, daß** beim Einführen des Instrumententeils (10) das Rastelement (7) durch ein Ende des Instrumententeils(10)längs einer Schräge (12) zwangsweise bewegt wird.

5. Kupplung nach Anspruch 4,
**dadurch gekennzeichnet, daß** die Schräge (12) konisch ausgebildet ist.

6. Kupplung nach Anspruch 4 oder 5,
**dadurch gekennzeichnet, daß** der Winkel (A), der durch die Achse (15) und die Schräge (12) definiert ist, größer ist als der Winkel (B), der durch die Achse (15) und den Einstich (11) definiert ist.

7. Kupplung nach einem der Ansprüche 1 bis 6,
**dadurch gekennzeichnet, daß** die Rasthülse (3) durch eine Rückstellkraft in Richtung ihrer Ausgangsposition gedrückt wird.

8. Kupplung nach ein Anspruch 7,
**dadurch gekennzeichnet, daß** die Rückstellkraft durch eine Feder, wie beispielsweise eine Schrauben-, Torsions- oder Druckfeder (5) bewirkt wird.

9. Kupplung nach einem der Ansprüche 1 bis 8,
**dadurch gekennzeichnet, daß** eine in der Drehhülse (1) angeordnete Aussparung (13), die z.T. durch die Schräge (12) begrenzt ist, über den Umfang der Drehhülse unterschiedlich große Querschnitte aufweist.

10. Kupplung nach einem der Ansprüche 8 oder 9,
**dadurch gekennzeichnet, daß** bei Drehung der Drehhülse (1) aus ihrer Ausgangslage das bzw. die Rastelemente (7) weiter in die Aussparung (13) reichen können und dadurch aus dem Einstich (11) des Griffs lösbar sind.

11. Kupplung nach einem der Ansprüche 8 bis 10,
**dadurch gekennzeichnet, daß** eine Rückstellkraft die Drehhülse (1) in Richtung ihrer Ausgangsposition drückt.

12. Kupplung nach der Anspruch 11,
**dadurch gekennzeichnet, daß** die Rückstellkraft durch eine Spiralfeder erzeugt wird.

13. Kupplung nach einem der Ansprüche 1 bis 12,
**dadurch gekennzeichnet, daß** mindestens ein lösbar fester Eingriff eines Stifts in ein Loch eine Drehung der Drehhülse (1) dann verhindert, wenn der Instrumententeil (10) mit der Kupplung verbunden ist.

14. Verfahren zum Verbinden eines Instrumententeils mit einem Schaftrohr unter Verwendung einer Kupplung nach einem der Ansprüche 1 bis 13,
**dadurch gekennzeichnet, daß**
- der Instrumententeil in das Schaftaufnahmeteil (2) geschoben wird und mit mindestens einem Rastelement (7) in Kontakt kommt,
- durch weiteres Schieben des Instrumententeils (10) das Rastelement (7) bis zu einer der Ausgangslage am entferntesten liegenden Stellung verschoben wird, und
- durch weiteres Schieben des Instrumententeils (10) sich das Rastelement (7) wieder zurück in Richtung der Ausgangsstellung und in den Einstich (11) des Instrumententeils (10) bewegt.

15. Verfahren nach Anspruch 14,
**dadurch gekennzeichnet, daß** die Entkupplung durch gleichzeitiges Drehen der Drehhülse (1) und Ziehen des Instrumententeils (10) geschieht.

16. Verwendung einer Kupplung nach einem der Ansprüche 1 bis 15 für zerlegbare Rohrschaftinstrumente oder für endoskopische Instrumente, die in einen Schaft eingesetzt sind.

17. Kupplung mit
- einer Rasthülse (3), die als eine Kugelhülse ausgebildet ist, die zwischen einer Drehhülse (1) und einem Schaftaufnahmeteil (2) angeordnet ist und die wenigstens eine Kugel (7) als Rastelement aufnimmt, wobei die Kugel bzw. Kugeln (7) nach außen bewegbar sind,
- einer Drehhülse (1) zum Lösen der Kupplung durch Drehung, die eine Aussparung (13) über ihren Umfang aufweist, die zumindestens zum Teil durch eine Schräge (12) begrenzt ist und unterschiedlich weit in die Drehhülse hineinreicht, wobei
- die Rasthülse (3) in Richtung der Längsachse der Kupplung verschiebbar ist und wenigstens teilweise in Kontakt mit der Drehhülse (1) ist, und wobei
- die Kugel bzw. die Kugeln (7) sowohl in Richtung der Längsachse der Kupplung mit der Rasthülse (3) mitbewegt werden und zusätzlich in etwa rechtem Winkel zur Längsachse der Kupplung bewegbar sind, wobei die Auslenkung der jeweiligen Kugel (7) durch die Schräge (12) und die im etwa rechten Winkel zur Längsachse angeordnete Ausdehnung der Aussparung (13) begrenzt ist.

18. Kupplung nach Anspruch 17,
**dadurch gekennzeichnet, daß** sie zur Verbindung eines Instrumententeils (10) oder eines Schaftrohres (9) dient und daß an einem Ende des Instrumententeils (10) bzw. des Schaftrohrs (9) mindestens ein Einstich (11) vorgesehen ist, in die die Kugel bzw. die Kugeln (7) nach dem Aufstecken greifen.

19. Kupplung nach Anspruch 18,
**dadurch gekennzeichnet, daß** die Verbindung durch Rückstellkräfte von mindestens zwei Federn oder durch formschlüssige Stifte hergestellt wird.

## Claims

1. Coupler for tubular-shaft instruments for releasable fixed connection of an instrument part (10) with a shaft tube (9), wherein the instrument part (10) on an outer surface (16) on an end (14) reaching up to the coupler or said shaft tube (9) is provided with at least one neck (11), and wherein the coupler is adapted to be mounted on said shaft tube or said instrument part, including
- a rotational shaft (1) for releasing the coupler by rotation, which is disposed around a shaft-receiving element (2), wherein
- a locking sleeve (3) mobile in an axial direction that is defined by the longitudinal axis (15) of said shaft tube (9), which is disposed between said rotating sleeve (1) and said shaft-receiving element (2) and which receives at least one locking element (7) that is movable to the outside, and
- wherein the coupler connects said instrument part (10) to said shaft tube (9) by locking engagement of said locking element or elements (7) with said neck or necks (11).

2. Coupler according to Claim 1,
**characterised in that** said or each neck (11) has a conical configuration.

3. Coupler according to Claim 1 or 2,
**characterised in that** said mobile locking sleeve (3) is a spherical sleeve while said locking element or elements (7) is/are spheres (7).

4. Coupler according to any of the Claims 1 to 3,
**characterised in that** when said instrument part (10) is inserted said locking element (7) is forcibly moved by one end of said instrument part (10) along an inclined plane (12).

5. Coupler according to Claim 4,
**characterised in that** said inclined plane (12) has a conical design.

6. Coupler according to Claim 4 or 5,
**characterised in that** the angle (A) that is defined by the axis (15) and said inclined plane (12) is wider than the angle (B) defined by said axis (15) and said neck (11).

7. Coupler according to any of the Claims 1 to 6,
**characterised in that** said locking sleeve (3) is pressed by a restoring force in the direction towards its initial position.

8. Coupler according to Claim 7,
**characterised in that** the restoring force is created by a spring such as a helical spring, a torsion spring or a compression spring (5).

9. Coupler according to any of the Claims 1 to 8,
**characterised in that** a recess (13) provided in said rotating sleeve (1), which is partly defined by said inclined plane (12), presents different cross-sections along the periphery of said rotating sleeve.

10. Coupler according to any of the Claims 8 or 9,
**characterised in that** when said rotating sleeve (1) is rotated out of its initial position said locking element or elements (7), respectively, can reach farther into said recess (13) while being releasable from said neck (11) in said handle.

11. Coupler according to any of the Claims 8 to 10,
**characterised in that** a restoring force presses said rotating sleeve (1) in the direction towards its initial position.

12. Coupler according to Claim 11,
**characterised in that** the restoring force is generated by a spiral spring.

13. Coupler according to any of the Claims 1 to 12,
**characterised in that** at least one releasable locking engagement of a pin in a hole prevents said rotating sleeve (1) from rotation when said instrument part (10) is connected to the coupler.

14. Method of connecting an instrument part to a shaft tube, using a coupler according to any of the Claims 1 to 13,
**characterised in that**
- said instrument part is pushed into said shaft-receiving element (2) and comes into contact with at least one locking element (7),
- under continued pushing action on the instrument part, the locking element is displaced up to a position most remote relative to the initial position,
- and under a continued further pushing action on the instrument part, the locking element returns again in the direction towards its initial position and into the neck on the instrument part.

15. Method according to Claim 14,
**characterised in that** decoupling is carried out by simultaneous rotation of said rotating sleeve (1) and drawing of said instrument part (10).

16. Application of a coupler according to any of the Claims 1 to 15 for tubular-shaft instruments adapted for disassembly or for endoscopic instruments inserted into a shaft.

17. Coupler comprising
- a locking sleeve (3) configured as spherical sleeve that is disposed between a rotating sleeve (1) and a shaft-receiving element (2) and receives at least one sphere (7) as locking element, wherein said sphere or spheres (7), respectively, are adapted for outward movement,
- a rotating sleeve (1) for releasing the coupler by rotation, which comprises a recess (13) along its periphery, which is defined at least partly by an inclined plane (12) and projects into said rotating sleeve over different distances, wherein
- said locking sleeve (3) is adapted for displacement in the direction of the longitudinal axis of the coupler and contacts said rotating sleeve (1) at least partly, and
- wherein said sphere or spheres (7), respectively, are adapted for being driven, together with said locking sleeve (3), in the direction of the longitudinal axis of the coupler and additionally for movement approximately at a right angle relative to the longitudinal axis of the coupler, with the deflection of the respective sphere (7) and the extension of said recess (13), which is disposed approximately at a right angle relative to the longitudinal axis, being limited by said inclined plane (12).

18. Coupler according to Claim 17,
**characterised in that** it serves to connect an instrument part (10) or a shaft tube (9), and that on one end of said instrument part (10) or said shaft tube (9), respectively, at least one neck (11) is provided for engagement of said sphere or spheres (7), respectively, therein after attachment.

19. Coupler according to Claim 18,
**characterised in that** the connection is established by restoring forces of at least two springs or by positive-locking pins.

## Revendications

1. Coupleur pour instruments à manche tubulaire pour la fixation relâchable d'une partie d'instrument (10) à un tube de manche (9), dans lequel la partie d'instrument (10) sur une surface extérieure (16) sur une extrémité (14), qui s'étend jusqu'au coupleur ou ledit tube de manche (9), est muni d'au moins un col (11), et dans lequel le coupleur est apte à être monté sur ledit tube de manche ou ladite parie d'instrument, comprenant
- une manche rotative (1) pour dégager le coupleur par rotation, qui est disposée autour d'un élément récepteur de la manche (2), dans lequel
- une manche de verrouillage (3) mobile in en sens axial, qui est défini par l'axe longitudinal (15) dudit tube de manche (9), qui est disposé entre ladite chemise rotative (1) et ledit élément récepteur de la manche (2) et qui reçoit au moins un élément de verrouillage (7), qui est mobile vers l'extérieur, et
- dans lequel le coupleur relie ladite partie d'instrument (10) audit tube de manche (9) par prise de verrouillage dudit élément ou desdits éléments de verrouillage (7) avec ledit col ou lesdits cols (11).

2. Coupleur selon la revendication 1,
**caractérisé en ce que** ledit ou chaque col (11) a une configuration conique.

3. Coupleur selon la revendication 1 ou 2,
**caractérisé en ce que** ladite manche de verrouillage mobile (3) est une manche sphérique, pendant que ledit élément ou lesdits éléments de verrouillage (7) est/sont des sphères (7).

4. Coupleur selon une quelconque des revendications 1 à 3,
**caractérisé en ce que** quand ladite partie d'instrument (10) est insérée, ledit élément de verrouillage (7) est déplacé à force par une extrémité de ladite partie d'instrument (10) le long d'une pente (12).

5. Coupleur selon la revendication 4,
**caractérisé en ce que** ladite pente (12) a une configuration conique.

6. Coupleur selon la revendication 4 ou 5,
**caractérisé en ce que** l'angle (A), qui est défini par l'axe (15) et ladite pente (12) est plus large que l'angle (B) défini par ledit axe (15) et ledit col (11).

7. Coupleur selon une quelconque des revendications 1 à 6,
**caractérisé en ce que** ladite manche de verrouillage (3) est pressée par une force de remise en un sens vers sa position de départ.

8. Coupleur selon la revendication 7,
**caractérisé en ce que** la force de remise est créée par un ressort comme un ressort hélicoïdal, un ressort de torsion ou un ressort de compression (5).

9. Coupleur selon une quelconque des revendications 1 à 8,
**caractérisé en ce qu'**un évidement (13) disposé dans ladite chemise rotative (1), qui est défini en partie par ladite pente (12), présente des coupes transversales différentes le long de la périphérie de ladite chemise rotative.

10. Coupleur selon une quelconque des revendications 8 ou 9,
**caractérisé en ce que** quand ladite chemise rotative (1) est tournée en dehors de sa position de départ, ledit élément ou respectivement lesdits éléments de verrouillage (7) peuvent s'étendre plus loin dans ledit évidement (13), en étant dégageable dudit col (11) dans ladite poignée.

11. Coupleur selon une quelconque des revendications 8 à 10,
**caractérisé en ce qu'**une force de remise presse ladite chemise rotative (1) en un sens vers sa position de départ.

12. Coupleur selon la revendication 11,
**caractérisé en ce que** la force de remise est engendrée par un ressort en spirale.

13. Coupleur selon une quelconque des revendications 1 à 12,
**caractérisé en ce que** la prise de verrouillage relâchable d'au moins une goupille dans un trou empêche une rotation de ladite chemise rotative (1), quand ladite partie d'instrument (10) est raccordé au coupleur.

14. Procédé de raccorder une partie d'instrument à un tube de manche, par l'emploi d'un coupleur selon une quelconque des revendications 1 à 13,
**caractérisé en ce que**
- la partie d'instrument est poussée dans l'élément récepteur de la manche et entre en contact avec au moins un élément de verrouillage,
- sous un effet de poussée en continu sur la partie d'instrument, l'élément de verrouillage se déplace jusqu'à une position la plus loin, relativement à la position de départ,
- et sous un effet plus continu sur la partie d'instrument, l'élément de verrouillage retourne encore en sens vers la position de départ et dans ledit col (11) de ladite partie d'instrument (10).

15. Procédé selon la revendication 14,
**caractérisé en ce que** le découplage se fait par une rotation de ladite chemise rotative (1) et une traction simultanée sur ladite partie d'instrument (10).

16. Emploi d'un coupleur selon une quelconque des revendications 1 à 15 pour instruments à manche tubulaire aptes à être démonté ou pour instruments endoscopiques insérés dans une manche.

17. Coupleur comprenant
- une manche de verrouillage (3) configurée comme manche sphérique, qui est disposée entre une chemise rotative (1) et un élément récepteur de la manche (2) et qui reçoit au moins une sphère (7) comme élément de verrouillage, ladite sphère ou respectivement lesdites sphères (7) étant mobiles à l'extérieur,
- une manche rotative (1) à dégager le coupleur par rotation, qui comprend un évidemment (13) le long de sa périphérie, qui est définie, au moins en partie, par une pente (12) et s'enfonce dans ladite chemise rotative sur des distances différentes, dans lequel
- ladite manche de verrouillage (3) est capable de se déplacer en sens de l'axe longitudinal du coupleur et entre en contact avec ladite chemise rotative (1) au moins en partie, et
- dans lequel ladite sphère ou respectivement lesdites sphères (7) est/sont apte(s) à être entraîné, ensemble avec ladite manche de verrouillage (3), en sens de l'axe longitudinal du coupleur et de plus à un mouvement environ à un angle droit relativement à l'axe longitudinal du coupleur, à la déviation de la sphère respective (7) et l'étendue dudit évidement (13), qui est disposé environ à un angle droit relativement à l'axe longitudinal, étant limité par ladite pente (12).

18. Coupleur selon la revendication 17,
**caractérisé en ce qu'**il sert à raccorder une partie d'instrument (10) ou un tube de manche (9), et **en ce qu'**à une extrémité de ladite partie d'instrument (10) ou respectivement dudit tube de manche (9), au moins un col (11) est formé pour la prise de ladite sphère ou respectivement desdites sphères (7) après le montage.

19. Coupleur selon la revendication 18,
**caractérisé en ce que** la connexion se fait par des forces de remise d'au moins deux ressorts ou moyennant des goupilles crabotées.
